# EUROPEAN PATENT APPLICATION

(11) **EP 4 136 974 A1**
(43) Date of publication of application: **22.02.2023**
(21) Application number: 21192432.9
(22) Date of filing: 20.08.2021
(51) Int. Cl.: A01N 63/40, A61L 27/34, A61L 31/10, A61L 31/16

(54) **PLASMA TREATMENT PROCESS AND APPARATUS THEREFOR**

(71) Applicant: Fixed Phage Limited, Glasgow G20 0SP (GB)
(72) Inventor: The designation of the inventor has not yet been filed
(74) Representative: Eyre, David Edward

(57) **Abstract**

The present invention relates to a method for producing products with molecules or macromolecules attached thereto and apparatus for carrying out this method. The method comprises the steps of: (a) generating a plasma at a location separated from the substrate; and (b) contacting the substrate exposed to the plasma with the biological macromolecule. Suitably the macromolecule is a bacteriophage. Thus, products of methods of the invention are for prevention and amelioration of bacterial contamination of the product or materials in contact with said product or products.

## Description

### Field of the Invention

The present invention relates to methods and apparatus for producing products with molecules or macromolecules, particularly biological macromolecules, attached thereto. Such macromolecules include bacteriophage. Thus, methods and products of the invention are for prevention and amelioration of bacterial contamination of materials put in contact or association with said products.

### Background to the invention

Bacteriophages or phages are viruses that specifically infect and kill bacteria, and which were first identified in 1915. As part of their life cycle, they infect and kill the bacterial host, releasing progeny which can then go on to infect other hosts. This ability to kill bacteria efficiently led to the suggestion that they could be used therapeutically, to treat bacterial infections. This technique, known as phage therapy, has a long history of peer reviewed publications demonstrating efficacy in both humans and animals. However, phages are biological entities and are therefore subject to inactivation by common environmental stresses, such as temperature, UV and pH. Phage-compatible formulations can increase stability and are being developed to increase the usability of phage products, with examples including gels, creams, encapsulation, and others. One method which has been proposed to stabilise phages for commercial deployment is the use of immobilization.

Immobilization refers to the chemical, physio-chemical or electrostatic binding of molecules to a surface, either directly or through the use of linkers. Immobilisation has been used to attach various biomolecules, including bacteriophages, to surfaces, with most research in the field of phage immobilization being carried out for the development of pathogen biosensors. Immobilization can localise and retain phages at specific locations and phages have been attached to various substrates by several mechanisms but usually indirectly, through the use of linkers. In the case of tailed phages, a 'tail-up' orientation is very much preferred to facilitate binding to bacteria and DNA injection. That is, attachment may be via the capsid or head portion of the bacteriophage thus leaving the tail, the part of the bacteriophage that contacts and binds to the target bacteria, pointing away from the substrate to facilitate binding to bacteria and DNA injection. Another factor that affects the ability of immobilized phages to infect their target bacteria is coating density and efficiency. Being able to consistently apply phages in a uniform density over the surface is desirable, as it allows for reproducibility, as well as the fact that phage clustering and non-uniform immobilization has been observed to hinder efficient bacteriophage function.

The applicants have previously shown that phages can be covalently attached to substrates by a direct application of energised plasma as exemplified through the use of corona discharge. Corona discharge is a stream of charged particles such as electrons and ions that is accelerated by an electric field. It is generated when a space gap filled with air or other gases is subjected to a sufficiently high voltage to set up a chain reaction of high-velocity particle collisions with neutral molecules resulting in the generation of more ions. Corona discharge has been applied to treat the surface of plastics to render them adherable and to increase wettability. Corona discharge takes place at atmospheric pressure. This is in contrast to low temperature (or cold) plasma that requires vacuum. The direct application of energy which generates plasma to a surface, is known as a primary plasma. Contrastingly, secondary plasma results from the spread and cascade of energised particles away from the high-energy source used for plasma generation.

Treating surfaces with primary (or direct) plasma or corona discharge imparts a high amount of energy to the surface which creates free radicals and other highly charged moieties, which allows phages or other biomolecules to covalently bind. Coronas are widely used as chemical reactors for surface treatment. Depending on the material to be treated and the desired surface properties, the optimal corona gas composition, discharge parameters, and operating procedure may be very different. An industrially very important application is the surface treatment of polymers, in particular to increase their wettability and adhesion to facilitate printing, painting, sealing, and coating. For example, if a plastic film is passed between the high-potential electrode and a grounded electrode, some of the ionized gas particles will undergo chemical reactions with the plastic surface, introducing reactive groups to the surface and increasing surface roughness.

Corona discharge is a means to generate plasma, the direct application of which the applicants have shown to effectively immobilise phages to substrates. Plasma, which is sometimes referred to as the fourth state of matter, is an at least partially ionised gaseous medium, made of excited, unstable and ionised atoms and molecules which emit visible and UV radiation. When matter is continually supplied with energy, its temperature increases and it typically transforms from a solid to a liquid and, then, to a gaseous state. Continuing to supply energy causes the matter to undergo a further change of state in which neutral atoms or molecules of the gas are broken up by energetic collisions to produce negatively charged electrons and positive or negatively charged ions. Other species generated in a plasma include high energy non-charged particles such as gas molecules in excited states, metastable compounds, molecular fragments and or radicals. The plasma is electrically neutral and therefore contains positive ions, negative ions and electrons in amounts such that the sum of their charges is zero. A plasma phase is obtained in the laboratory by subjecting a pure gas or a gaseous mixture to external excitation, which is most generally electrical.

The term "plasma" covers a wide range of systems whose density and temperature vary by many orders of magnitude. Some plasmas, commonly known as thermal equilibrium plasmas are very hot and all their microscopic species (ions, electrons, etc.) are in approximate thermal equilibrium, the energy input into the system being widely distributed through atomic/molecular level collisions; examples include flame-based plasmas. Flame based plasmas operate at high gas temperature and are oxidative by nature which means they have significant limitations when applied to deposition processes. In such high temperature gases it is impossible to maintain the chemical structure and/or functionality of the precursor in the deposited coatings. Furthermore, the high process temperatures involved are incompatible with heat sensitive substrates.

Other plasmas, however, particularly those at low pressure (e.g. 100 Pa) where collisions are relatively infrequent, have their constituent species at widely different temperatures and are called "non-thermal equilibrium" plasmas. In non-thermal equilibrium plasmas, free electrons are very hot with temperatures of many thousands of Kelvins (K) whilst neutral and ionic species remain cool. Because the free electrons have almost negligible mass, the total system heat content is low and the plasma operates close to room temperature thus allowing the processing of temperature sensitive materials, such as plastics or polymers, without imposing a damaging thermal burden.

While direct corona treatment (i.e. primary plasma) of surfaces is a well-understood and efficient means of treatment, it does have some disadvantages. Because it imparts a high level of energy to the surface, labile surfaces, such as paper and fine fibres, are liable to be damaged by the treatment and are therefore difficult to treat effectively. Furthermore, because the treated material generally needs to be passed through the gap between the electrode and the ground, substrates must be smaller than a few centimetres in height and irregularly shaped objects are unevenly treated. While corona discharge is efficient, bridging larger gaps requires an exponential increase in power which rapidly becomes practically unachievable.

Several means of generating plasma are described in the art. One type of plasma is generally referred to as diffuse dielectric barrier discharge (one form of which can be referred to as an atmospheric pressure glow discharge Sherman, D.M. et al, J. Phys. D.; Appl. Phys. 2005, 38 547-554). This term is generally used to cover both glow discharges and dielectric barrier discharges whereby the breakdown of the process gas occurs uniformly across the plasma gap resulting in a homogeneous plasma across the width and length of a plasma chamber. (Kogelschatz, U. 2002 "Filamentary, patterned, and diffuse barrier discharges" IEEE Trans. Plasma Sci. 30, 1400-8) These may be generated at both vacuum and atmospheric pressures. In the case of atmospheric pressure diffuse dielectric barrier discharges, gases including helium, argon or nitrogen are utilised as process gases for generating the plasma and a high frequency (e.g.> 1 kHz) power supply is used to generate a homogeneous or uniform plasma between the electrodes at atmospheric pressure.

Atmospheric pressure plasmas offer industry open port or perimeter systems providing free ingress into and exit from the plasma region by e.g. webbed substrates and, hence, on-line, continuous processing of large or small area webs or conveyor-carried discrete workpieces. Throughput is high, reinforced by the high species flux obtained from high pressure operation. Many industrial sectors, such as textiles, packaging, paper, medical, automotive, aerospace, etc., rely almost entirely upon continuous, on-line processing so that open port/perimeter configuration plasmas at atmospheric pressure offer a new industrial processing capability.

WO 02/28548 describes a process to overcome the limitations to vacuum and some pulse type applications. By combining an atmospheric pressure plasma discharge, such as a diffuse dielectric barrier discharge, and an atomised precursor, a range of coatings may be deposited which retain the functionality of the precursor to a large degree. Using this technique, a controlled free radical polymerisation takes place, and the monomer structure is significantly retained.

Post discharge plasma systems have been developed to produce plasmas using gases passing between adjacent and/(or coaxial) electrodes at high flow rates. These gases pass through the plasma region defined by the shape of the electrodes and exit the system in the form of excited and/or unstable gas mixtures at around atmospheric pressure. These gas mixtures are characterized by being substantially free of electrical charged species, which may be utilized in downstream applications remote from the plasma region, i.e. the gap between the adjacent electrodes in which plasma is generated. This "atmospheric pressure post plasma discharge " (APPPD) has some of the physical characteristics of low-pressure glow discharge and APGD including, for example, glow, presence of active light emitting species and chemical reactivity. However, some clear and unique differences exist including the facts that APPPD has higher thermal energy, absence of boundary walls e.g. no electrodes, substantial absence of electrically charged species, large choice of gases and mixture of gases, large flow rate of gases. Systems of this type are described in US 5807615 , US 6262523 and WO 2005/039753.

Hot-Filament Chemical Vapour Deposition (HFCVD) is an alternate method for depositing polymeric coatings on substrates which, unlike plasma enhanced Chemical Vapour Deposition (PECVD), does not use a plasma to initiate a free radical based CVD process but uses a heated filament to initiate a thermal CVD reaction. Recent work using HFCVD has shown that the addition of free radical initiators to a monomer vapour can result in increased retention of the monomer functionality in the resulting polymerised coating (for example, Gleason etal, Langmuir, 2002, 18, 6424).

The use of catalysts to initiate free-radical polymerisation reactions is a well-known and commonly used technique. For example, WO 0034341 describes a heterogeneous catalyst for the polymerisation of olefins. U.S. Pat. Nos. 5,064,802 , 5,198,401 , and 5,324,800 also describe selective catalysts for olefin polymerisation. U.S. Pat. No. 2,961,245 describes the polymerisation of cyclotrisiloxane containing fluorinated hydrocarbon radicals, in the presence of a homogeneous initiator such as perfluoroalkanesulphonic acid and of linear organosiloxanes with triorganosilyl ends that are used as chain-blocking agents. A fluorinated silicone oil is thus obtained, after devolatilization. The catalyst is optionally removed by distillation or washing. EP 0822240 describes a coating resin composition formed from an acrylate, organosilane and a curing catalyst.

In addition, US 7,250,195 discloses generating a corona discharge which is introduced into a vacuum chamber for deposition of biomaterial (including amino acids and proteins, but not bacteriophage) onto a substrate. WO 2014/134297 discloses methods of covalently attaching bacteriophage to surfaces by forming carboxylic acid residues on the surface of the substrate to which the bacteriophage can covalently bond.

The use of both primary and secondary plasma for surface modification, including use of the plasma generation methods described above, is well established. Typically, the substrate to be coated is placed within a vessel, and a plasma is formed. Introducing a coating-molecule into this plasma will then give rise to a plasma polymerisation reaction and lead to the non-covalent deposition of a polymer onto the substrate. Many examples of such treatment are known in the art; for example, US 5,876,753 discloses a process for attaching target materials to a solid surface which process includes affixing carbonaceous compounds to a surface by low power variable duty cycle pulsed plasma deposition, and EP 0896035 discloses a device having a substrate and a coating, wherein the coating is applied to the substrate by plasma polymerisation of a gas comprising at least one organic compound or monomer. Similarly, WO 00/20130 describes a process for depositing a hydrophobic coating onto a solid substrate by exposing the substrate to a plasma containing a suitably substituted alkyne. EP 0095974 describes a process for the polymerisation of pre-prepared supported film which have been applied onto a substrate surface prior to the application of a plasma in a vacuum. Radical initiators may be used in the pre-prepared film as sensitizers. Similarly, WO 2003/089479 describes a process in which a composition including both a free-radical polymerisable compound and a photolatent compound, which may be a free-radical photoinitiator, is applied in a liquid form onto a three-dimensional substrate surface and is subsequently plasma treated in a vacuum chamber. Charles W. Paul, Alexis T. Bell and David S. Soong, Macromolecules 1985, 18, 2312 - 2318, discuss the initiation of methyl methacrylate polymerisation with a free-radical initiator. The free-radical initiator is produced in a vacuum glow discharge process. US3962486 describes a method of plasma spraying cured thermoset coatings by propelling resinous particles with sufficient force and velocity at elevated temperatures e.g. 350F (175°C) towards a substrate to cause impact and to cause the particles to flow and coat the substrate surface.

WO97/38801 describes a method for the molecular tailoring of surfaces which involves the plasma deposition step being employed to deposit coatings with reactive functional groups, which groups substantially retain their chemical activity on the surface of a solid substrate, using pulsed and continuous wave plasma. WO03/084682 describes a method for producing a protective coating composition by activating either a polymerisable organic acid or acid anhydride and/or a polymerisable organic base using a soft ionisation process and then depositing the activated monomers onto a substrate surface.

US7250195 describes a method allowing the deposition of biological molecules on substrates using plasma but does not describe the need for the rapid application of those biological molecules to enable long-term stability. Furthermore, US7250195 describes biomolecules being treated with a plasma rather than the substrate being treated with the plasma.

Methods for treatment of powders with vacuum plasma are described in the art. For example, WO2010142953 describes a method of treating small particles, particularly carbon nanotubes, using a rotating drum and a central electrode that generates plasma via a glow discharge, but this is solely for modifying the surface chemistry of these particles and does not provide a mechanism for the attachment of molecules or biomolecules to these particles, but rather provides a method for the disaggregation of the carbon nanotubes. WO2012076853 provides a similar method for treating small particles in the presence of conductive contact bodies but also does not provide a method for attaching molecules to the treated graphitic particles.

One application of the use of plasma-mediated immobilisation is the immobilisation of bacteriophages to various surfaces. International patent application WO 03/093462 discloses that immobilised bacteriophages can be covalently attached to a substrate using chemical methods or an electrical discharge, making it impossible for them to diffuse freely, but retaining their ability to infect cells, thus subsequently causing bacterial lysis and release of free bacteriophage. In WO 2007/072049 those methods were developed to the use of a pulsed field corona discharge; particles activated by the discharge were dropped into bacteriophage solutions. Similarly, in WO 2012/175749 bacteriophage solutions were combined with activated seeds. Therapeutic applications of bacteriophage are known. WO 03/093462 discloses methods for the immobilisation of viruses, in particular bacteriophages, while retaining their biological activity for use as antibacterial agents. Given that the natural environment of bacteriophages is aqueous it has been widely assumed that stability towards dehydration as disclosed in WO 03/093462 tends towards the natural stability in aqueous media. WO2012/175749 describes small particles with bacteriophage attached thereto that are suitable for combining with foodstuffs to ameliorate bacterial contamination thereof as disclosed in. WO2014/049008 describes small particles with bacteriophage attached thereto that are particularly suitable for treating or preventing bacterial infections. Wang et al (2016)¹ have shown that low pressure plasma treatment, a form of electrical discharge treatment, for 1 minute in an oxygen atmosphere enhanced the adhesion of bacteriophages to surfaces. Such treatment of surfaces, to allow the stable binding of molecules or macromolecules (including phage), is referred to as "activation". Surface activation by corona discharge or other plasma generating electrical methods is well known (see e.g. Barton et al (2003)²; Canavan et al (2006)³; Recek et al (2014)⁴; Kerkeni et al (2013)⁵, and others). It is well known in industrial practice, for example in printing, that such electrical treatments result in increased hydrophilicity of surfaces (wettability) and improved adhesion. Such increased adhesion due to greater hydrogen bonding differs from the use of rapid contact between the electrically activated surfaces and bacteriophages described in the above patents to achieve covalent bonding.

Notably, the methods hitherto described to covalently attach phages to substrates have relied on primary plasma, i.e. the direct application of plasma to a surface, where the substrate is generally passed between the electrode and the ground, which provides the most energy to the surface. This is the only method that has been proposed to allow covalent bonding of phages to a substrate, due to the high levels of energy required to sufficiently activate the surface. Covalent immobilization represents the most permanent and irreversible form of attachment, demonstrated by the ability of covalently immobilized phages to remain bound to substrate even after prolonged exposure to shear or sonication forces. Studies have also found covalent-based approaches allow for increased binding efficiency, with one study reporting a 37-fold increase in binding efficiency compared to unaided physical adsorption (Hirsch et al., 2010)⁶. In order for phages to be covalently attached to the surface of treated materials, they must be applied to the surface rapidly (< 1 minute but ideally as soon as possible), so that the surface is still highly reactive, allowing covalent bonds to form. Covalent attachment is required for the maximum stability benefits of immobilization to be achieved.

The use of various secondary plasmas for surface treatment is well understood in the art, this is limited to surface modifications to (and non-covalent binding of substrate to) a surface. While this method of coating is sufficient for most applications it does not address the unique requirements of covalent immobilisation of large biomolecules, such as bacteriophages, which is stabilisation of those biomolecules through formation of covalent bonds between the substrate and the surface. Previously, where covalent stabilisation of biomolecules has been demonstrated this has been by the direct application of plasma/corona to the substrate (e.g. exposed to a primary plasma by passing the substrate being activated between the electrode and the ground) and it was assumed that the high-energy conditions required to allow covalent bond formation required this direct treatment, but this had the limitations of restricting the shape of object that could be treated and requiring careful tuning of the process to treat heat-sensitive objects. Both papers and some powders have been shown to be sensitive to burning when exposed directly to the energies needed to activate substrates to allow covalent attachment of biomolecules.

A further advantage of the use of secondary plasmas is that they have a greater diffusion potential than primary plasmas, so that substrates with complex microarchitecture, e.g. pores, or other surface irregularities, are more evenly treated.

There is therefore the need to develop a method of treating materials with plasma indirectly, to allow the immobilisation and stabilisation of bacteriophages on heat-labile substrates or those which are difficult to treat reproducibly with the standard platform or belt-fed apparatus.

### Object of the Invention

The object of the current invention is to provide methods for the treatment of substrates, including powders, particles and solid substrates to allow immobilisation of a biological macromolecule, e.g. bacteriophage, on a substrate.

### Summary of the Invention

Accordingly, the invention provides a method of covalently attaching biological macromolecule to a substrate comprising the steps of:
(a) generating a plasma at a location separated from the substrate; and
(b) contacting the substrate exposed to the plasma with a biological macromolecule.

This method yields the following advantages:
the plasma used for activation may be initially generated at a site distinct from the object being treated;
a biological macromolecule may be applied to the substrate by application of carrier fluid, such as a liquid or gas;
the biological macromolecule is applied to the surface rapidly (< 1 minute) after plasma activation to enable immobilisation (i.e. covalent attachment). Thus the activation of the surface may be sufficiently energetic to allow the permanent chemical bonding, preferably covalent bonding (immobilisation) of biomolecules, including biological macromolecules, e.g. bacteriophages; and/or
the biological macromolecule may retain complete or partial activity after immobilisation.

The object provides a substrate for the attachment of the molecule or macromolecule thereto. Accordingly, the terms object and substrate are used interchangeably herein.

The molecule or macromolecule may be a biomolecule or a biological entity. Biomolecules or biological molecules are molecules present in organisms that are active in one or more biological processes (e.g. bacteriophage infection, immune response, cell division, morphogenesis, or development) Biomolecules include large macromolecules (or polyanions) such as proteins, carbohydrates, lipids, and nucleic acids, as well as small molecules such as primary metabolites, secondary metabolites and natural products. A more general name for this class of material is biological materials. Thus the biomolecule may be an antibody, cytokine or other signalling molecule. Alternatively, or in addition the molecule or macromolecule may be a biological entity such as multimeric protein or antibody, or a virus. Preferably the macromolecule is a virus and most preferably the macromolecule is a bacteriophage.

Thus, bacteriophage immobilised to a substrate according to the present invention, may be utilised to fight strain-specific bacterial infections as a "bactericide" by inducing selective killing of bacteria through cell lysis or as a "bacteriostatic agent" by inhibiting bacterial growth. Bacteriophage immobilised to a substrate may also be used as an antibacterial agent/disinfectant in order to "sterilise" bacterially contaminated material. Accordingly, preferably the bacteriophage retains infectivity when immobilised.

The term "substrate" is understood to mean any solid phase material to which a molecule or macromolecule may be immobilised following exposure of the substrate to a suitable electrical discharge. Said substrate may take many forms, for example made of a material commonly used in therapy/medicine. For example, nylon thread for use in surgery; plastics, lint or gauze material used to dress open wounds; microbeads, which can be ingested; adhesives such as cyanoacrylates; and/or biological substances such as collagen or hyaluronic acid. Said material may be edible food or feed particles, containing proteins and bulking material, such as cellulose or other plant-derived polymers. More preferably, the material may be a powder which could comprise nylon and any other polymer with amino or carboxyl surface groups, cellulose or other hydroxyl-containing polymer, polystyrene or other similar polymer, various plastics or microbeads including magnetic particles, clays such as kaolin and biological substances.

The substrate may be a textile. Textiles include natural and synthetic textiles. Textiles may be woven or non-woven. Textiles may be laminated with layers of the same or different textiles. Natural textiles include cotton, silk, denim, flannel, hemp, leather, linen, velvet, and wool. Synthetic textiles include nylon, polyester, acetate, acrylic, and rayon.

The term "activated/activating/activation" according to the present invention is understood to mean the activation of a substrate by reacting said substrate with various chemical groups (leaving a surface chemistry able to bind bacteriophage head or capsid groups). In this invention, activation of said substrate is achieved by indirect treatment with secondary plasma i.e. the plasma used is generated in a separate chamber and the substrate does not directly pass through the electrode. Contact between a molecule or macromolecule and the activated substrate leads to the formation of a covalent bond between the molecule or macromolecule and the substrate. Accordingly, use of the term "attaching" in the context of the present invention means covalent attachment.

Various forms of plasma generation are compatible with this invention, including those cited in the references given above and including but not limited to plasmas generated by electrical or microwave energy.

A fluid is a continuous, amorphous substance whose molecules move freely past one another and that has the tendency to assume the shape of its container. Thus a liquid or gas is a fluid substance.

A product of the method of the invention may be retreated according to the method of the invention.

Advantageously, the use of some secondary plasma (e.g. those that exist at close to ambient temperature) allows surfaces to be retreated with the process, as low temperature plasmas (<60°C) are compatible with retaining biological activity of the attached molecules.

The biological macromolecule may contact the substrate under conditions of reduced pressure or vacuum, typically at pressures of 1 to 100 mbar.

The plasma generated may be a vacuum plasma.

The method may further comprise the step of releasing the reduced-pressure condition or vacuum by the admission of an inert gas, preferably argon or nitrogen.

The plasma may be moved by a fluid to expose the substrate to the plasma, preferably the fluid is a gas, preferably exposure of the substrate to the plasma causes activation of the substrate.

In preferred use of the invention, the bacteriophage retains infectivity.

The plasma may be produced by the ionisation of air. Alternatively, the plasma may be generated in a background of inert gas e.g. nitrogen, neon, argon, or krypton, at up to 1 atmosphere.

The plasma may be produced by exposure of a gas to radiofrequency energy with a frequency of between 10 kHz and 50 Mhz.

The plasma may be produced using an electrode, using powers of between 10 and 10000 Watts.

The substrate may be exposed to the plasma for a duration of 1 and 100 seconds.

The substrate may remain in place between the exposure step and the contacting step. Thus preferably the exposing step is carried out within a chamber, more preferably the chamber is sealable most preferably the chamber is suitable for maintaining a vacuum or pressures lower than atmospheric pressure. Also preferred is the step of contacting the activated substrate with the substance to be attached thereto being carried out within the same chamber.

Biological macromolecules may be attached to substantially all surfaces of the substrate by use of the method of the invention.

A method according to any preceding claim, wherein no area of the substrate larger than 1 mm² remains without bacteriophage attached thereon.

The substrate may have surface features of less than 100 µm diameter.

The substrate may have surface indentations or pits of less than 100 µm.

The substrate may be a powder. Preferably the particles of the powder have diameters in the range of 0.5-1000 µm. The substrate may be a nanoparticle of size 1-500 nm in diameter. The diameters of the particles of the powder or the nano particles may be measured by means of laser diffraction.

The substrate may be a particle. The particles may be of a size less than or equal to 50 mm in diameter, preferably less than or equal to 20 mm in diameter. The particle may be substantially spherical. Suitably, the particles may be food pellets. The food pellets are suitable for consumption by humans and/or animals. Preferably the food pellets are suitable for use as animal feed. The pellets may preferably be in the range of 1 mm - 50 mm in height as measured from a surface they are lying or supported on. The pellets may be substantially spherical, cylindrical or spherocylindrical.

Suitable apparatus for laser diffraction measurement of particles and/or powders is supplied by Malvern Panalytical, Malvern, UK, e.g. the Mastersizer^{™} apparatus.

In the case of powders, particles or pellets, advantageously even coating with a plasma may be achieved by agitating the substrate in rotating or intermittently rotating drum. Thus the powder being treated or to be treated may be located in a rotatable drum. The rotatable drum may be rotated continuously or intermittently to agitate, tumble, or mix the powder. The rotation may be in one direction or be reciprocal motion.

Preferably the drum is rotated during either or both of the step of contacting the substrate exposed to the plasma with the biological macromolecule and moving the plasma to expose the substrate thereto.

In addition, the object may be a seed e.g. a plant seed. Seeds may be advantageously treated according to the invention because direct treatment of seeds with corona discharge affects germination times and interfere with standard distribution/planting times. Indirect treatment of seeds by plasma allows immobilisation of biomolecules to the surface of the seed without affecting germination times. Seeds suitable for treating according to the invention may be of a size less than or equal to 10 mm in diameter, preferably in the range of 0.2 to 5 mm.

The substrate may be a textile.

The textile may be woven or non-woven.

The substrate may comprise silk strands. Preferably the silk strands are of an average diameter 0.1 to 100 micrometers.

The substrate may be paper. Preferably, the paper is of an average gsm of less than 300 g/m².

The substrate may be a plastics material. The plastics material may be selected from: polypropylene, polypropylene homopolymer, polypropylene copolymer, polyethylene, low density polyethylene, high density polyethylene, polystyrene, expanded polystyrene; acrylic, acrylonitrile butadiene styrene, nylon (polyamide), acetal, polycarbonate, acrylate styrene acrylonitrile, styrene acrylonitrile, polyvinyl chloride, polyurethane, polyesters, polysulphone, polyphenylene sulphide, polyvinylidene fluoride, polyphenylene oxide, ethylene vinyl acetate. Preferably the plastics material is polyphenylene sulphide or expanded polystyrene.

The substrate may be a medical device. The medical device may be an orthopaedic joint replacement, or component thereof. The medical device may be a hip joint or knee joint. The medical device may be a dental implant. The medical device may be a stent, preferably a vascular stent.

The medical device may comprise or incorporate one or more of the substrate materials described herein.

The substrate may be an object with complex surface topography, such as an implant or catheter, where the greater penetration of secondary plasma allows more efficient treatment of the uneven surface. Furthermore, the object may contain surface features in the micron and sub-micron range which are more readily treated by the application of a secondary plasma, and preferentially by a vacuum plasma.

Thus the methods and apparatus herein are particularly advantageous for treating medical devices. Such devices may include:
syringes, cannulas, needles; catheters; coils, guidewire; medical tubing or hoses; endoscopic devices including laryngoscopes, laparoscopes, anascopes, proctoscopesarthroscopes, sinuscopes, dematoscopes, ophthalmoscopes, sigmoidscopes, otoscopesretinoscopes, or colposcopes; stethoscopes; speculums; medical scissors; forceps; single-use medical procedure trays and kits; ear syringes; ear wax removers; clinical swabs, applicators, specimen collectors, sponges, pads, cotton balls, or cotton rolls; orthopaedic supports, braces, wraps, shoes, boots, or pads; surgical sutures and staples; and removal kits; tourniquets; condoms; electrocardiography machines; cardiology ablation devices and accessories; cardiology balloon (extractor, retrieval); cardiac pacemaker; cardiopulmonary oxygenation devices; heart positioners, heart valves; dental equipment and supplies; bone graft matrix; dentures, crowns, moulds, orthodontics; dental instruments; dental and oral implants or devices; haemodialysis connection or tubing kits; implantable neurostimulator; umbilical cord clamps; ventilator, and/or tubing and/or accessories; contact lenses; cervical fusion kit; orthopaedic plates/screws, fixators, implants; surgical shunts; tissue stabilizers; surgical clips; surgical instruments; surgical instrument cases, trays, mats or tray liners, racks, covers, wraps, stands, holders stringers, or protectors; surgical stents; surgical linens, drapes, or covers; chest drains; blood transfusion equipment; surgical clean-up kits; wound drainage equipment; stockinettes; surgical mesh; electrosurgery devices and/or supporting equipment.

The substrate may be a material used in the manufacture of wearable items, such as clothes, shoes, scarfs, masks and similar, where the fibres used in manufacture are sensitive to the high temperatures of primary plasma application or where more efficient treatment may be obtained through the use of a secondary plasma.

Such items may include any of medical gowns, scrubs, aprons, uniforms, lab coats, and coveralls; only those without, integrated hoods, patient clothing including gowns, slippers, under pads, or undergarments, head or beard covers and nets, medical shoe and boot covers, surgical sleeve protectors, ventilated safety eye shields and goggles, disposable latex, nitrile, polyethylene, vinyl gloves/finger cots or other medical gloves, surgical face or dust masks.

The invention also provides an apparatus comprising covalently attaching a biological macromolecule to a substrate comprising:
(a) a treatment area or chamber;
(b) means for generating a plasma at a location separate from the treatment area or chamber;
(c) means for contacting objects in the treatment area or chamber with the biological macromolecule.

The apparatus may comprise means for moving the plasma to the treatment area or chamber.

The treatment area or chamber may comprise a rotatable drum. Preferably the rotatable drum is removable from the apparatus.

The rotatable drum may be sized to allow agitating, tumbling or mixing of powder located therein. Preferably, the rotatable drum is removable from the apparatus.

The apparatus may be for carrying out a method of the invention.

The generated plasma may be blown onto the substrate, with the substrate being moved through the blown plasma on a conveyor, such as a moving surface, preferably a belt, most preferably a continuous belt.

Advantageously, the plasma may be more efficiently applied to the substrate using a vacuum, by containing the substrate to be treated in a chamber from which air can be removed. In a further form of the invention said chamber may contain a drum which rotates, for example to enable the efficient treatment of powders and particles which are agitated during plasma addition. The plasma applied to the substrate may be applied either as the vacuum is formed or once the vacuum reaches a set target pressure.

Additionally, once a vacuum is formed around the substrate, but before the biological macromolecule is introduced thereto, an alternate gas may optionally be injected into the chamber to facilitate efficient binding of the macromolecule to the substrate. Such gasses or gas mixtures are preferentially noble or inert gases, preferentially argon or nitrogen, but may also be reactive, e.g. air or oxygen, in order to promote the formation of certain covalent bonds.

A further feature of the invention is the ability to sterilise materials prior to the addition of a biomolecule and to retain that sterility throughout the immobilisation process.

Plasma treatment is well-known in the art to sterilise substrates it is applied to (for example, Moisan etal., (2002). Pure Appl. Chem., Vol. 74, No. 3, pp. 349-358). This sterilisation of substrate may have applications, for example in the treatment of certain medical devices, where the ability to sterilise a substrate and to apply a biomolecule to that substrate in a contained chamber such that sterility is preserved. One example would be in the treatment of an implant for human use, where the implant could be treated in a chamber with plasma, which both activates and sterilises it, before the aseptic addition of bacteriophage(s). This would result in a sterile medical device, ready for direct implantation or packaging for distribution.

### Examples and Description of the Drawings

The invention is now illustrated in the following specific embodiments with reference to the accompanying drawings showing:-
- Figure 1:: Shows cellulose powder treated by indirect plasma with bacteriophages applied. Zones of clearing round the powder indicate where the bacteriophages have killed their bacterial target host. Figure 1a shows the extent of clearing in a bacterial lawn on a petri dish. Figure 1b shows a close-up of treated cellulose powder on a bacterial lawn with a zone of bacterial killing surrounding the powder particles.
- Figure 2:: shows photographs of cellulose powder that is visibly burned after exposure to the direct exposure to the electrode generating a plasma.
- Figure 3:: shows the result of exposure visible damage and burning of paper towels exposed to the electrical field generated by the corona discharge electrode.
- Figure 4:: shows a bar chart of the calculated activity of phage immobilised onto cellulose powder using vacuum plasma stored at room temperature (RT) and 40 °C. The chart shows that greater bacteriophage activity was observed in immobilised phage material compared to the adsorbed controls.
- Figure 5:: shows a photograph of bacteriophage immobilised onto heat-sensitive wound dressing materials using indirect vacuum plasma treatment. Zones of clearing of the bacterial lawn round the wound dressing materials indicate where the bacteriophages have killed their bacterial target host

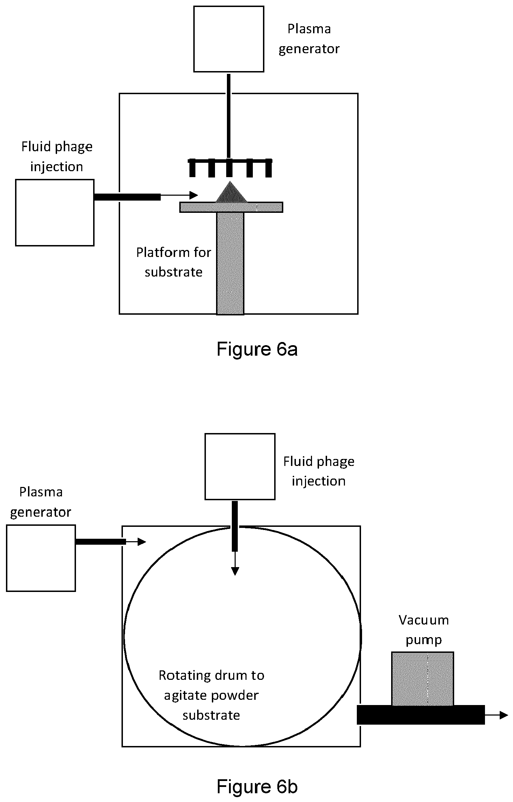

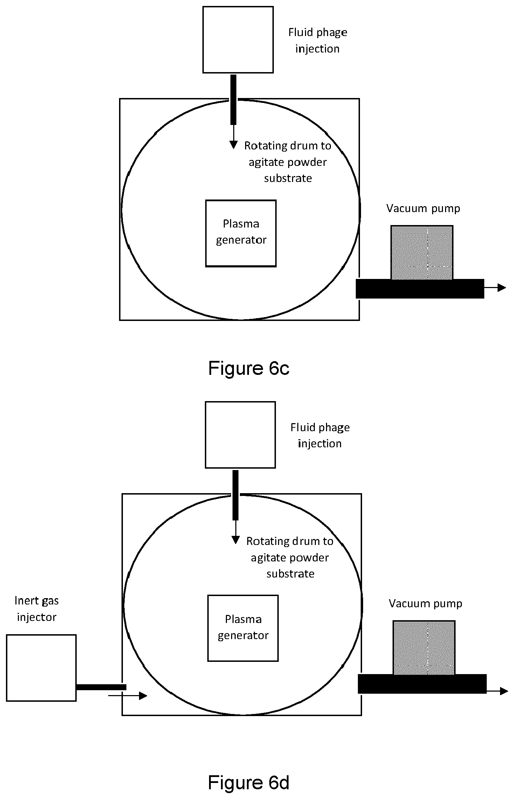

- Figure 6:: Schematic diagrams of apparatus for treating substrates with vacuum plasma. Preferably these apparatuses are for applying biomolecules for immobilisation in a single chamber.

### Example 1

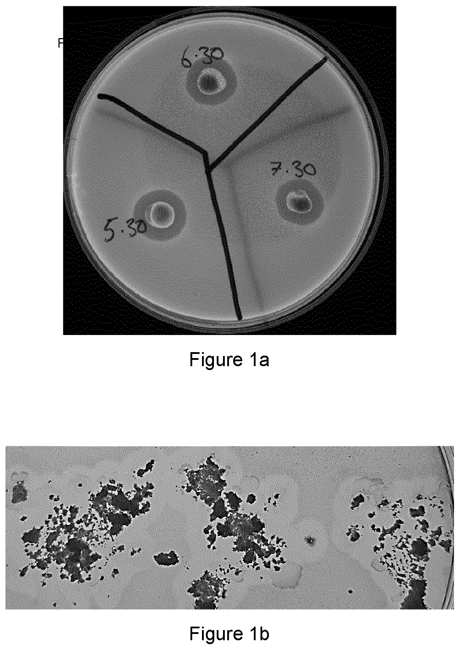

Cellulose powder was treated for 1 min at 200 Watts in a VacuTEC 2020 vacuum plasma machine. In order to capture the high-energy state that allows phage immobilisation after activation, plasma was evacuated from the chamber and phages sprayed onto the powder <30 seconds after plasma removal. After drying, powder was then spread or spotted onto a lawn of target bacteria to determine activity. In both cases, treated powder was shown to effectively kill the target bacteria, as demonstrated in Figure 1 by zones of clearing of target bacteria round the treated materials.

### Example 2

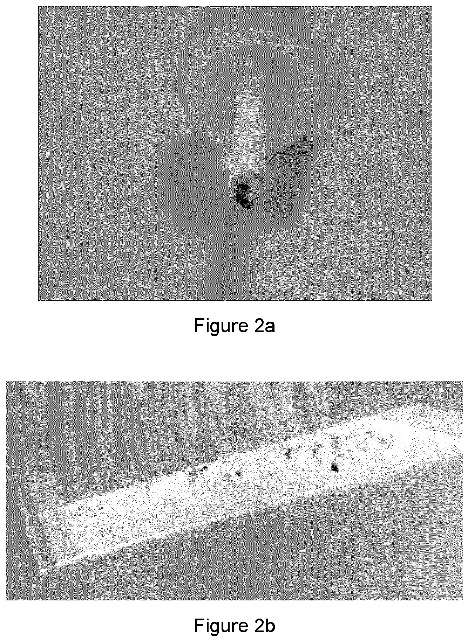

Demonstration that direct application of corona discharge to cellulose powder can results in damage (burning) to the powder (Figure 2a and 2b). Powder treated with indirect vacuum plasma is not damaged by the activation.

### Example 3

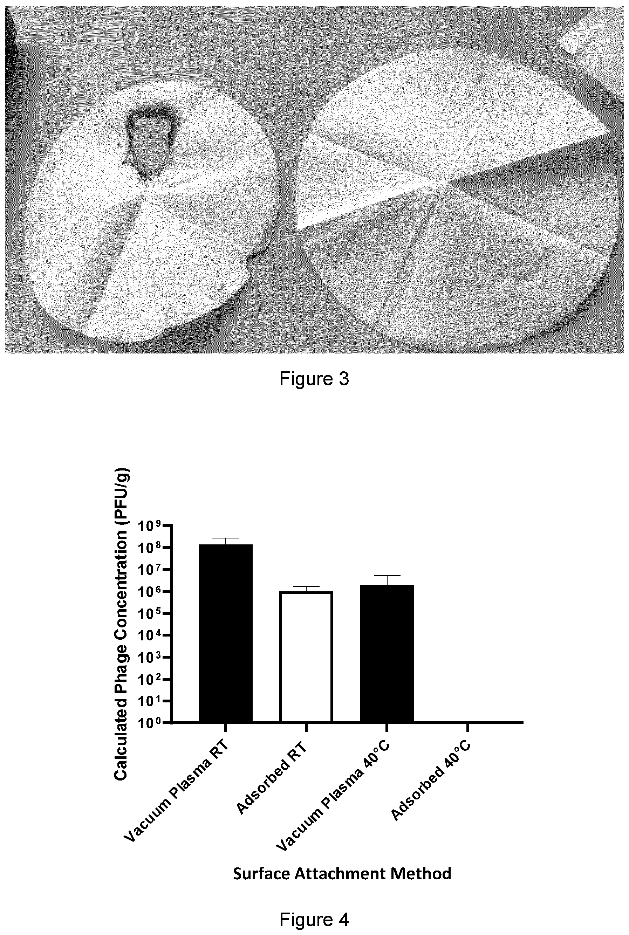

Example of paper towel treated with either direct corona discharge (Figure 3, left) or indirect vacuum plasma (Figure 3, right). For direct treatment the paper towel was passed through a direct 7.5 kW corona discharge, for vacuum plasma an identical towel was treated for 1 min in a 200 Watt plasma discharge. The direct treatment resulted in clear damage to the towel with scorching/burning visible on the surface, lower direct treatment voltages did reduce damage, but phage binding efficiency was also reduced.

### Example 4

Cellulose powder was exposed to 30 seconds of vacuum plasma treatment (200 Watts) at 0.2mbar. After treatment, bacteriophages were applied to the powder and a sample stored at ambient room temperature (RT) (22 °C) and 40 °C along with samples of powder with phage applied to the surface without exposure to plasma. Phage activity was measured after 18 days of incubation at each temperature and the results are shown in Figure 4. More phage activity was observed on the samples prepared using vacuum plasma than the adsorbed samples. No phage activity was detected on adsorbed samples stored at 40 °C.

### Example 5

To test the survival of phage under vacuum, a solution of ϕT4 and a sample of ϕT4 immobilised onto cellulose powder was divided into 2 samples. A sample of the phage solution and immobilised cellulose powder was added to a vacuum plasma chamber and exposed to an atmosphere of 2 millibar for 30 seconds without plasma activation. The phage concentration of the solutions and the immobilised phage was then assessed and compared to the phage concentration of the samples not exposed to vacuum and the results are shown in Table 1, below. No significant difference in phage activity was observed in phage solutions or material after exposure to the vacuum conditions.

**Table 1: Survival of bacteriophage exposed to a vacuum.**

| | **Non - vacuum control** | **Vacuum** |
|---|---|---|
| **Phage T4 solution** | 5x10⁷ PFU/ mL | 6x10⁷ PFU/ mL |
| **Phage T4 immobilised onto cellulose.** | 6x10² PFU/gram | 5x10² PFU/gram |

### Example 6

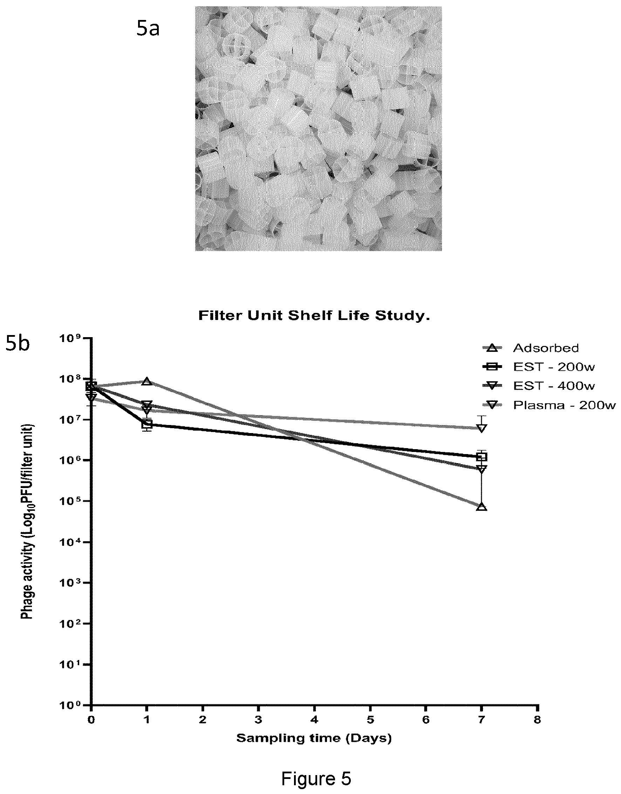

K1 biofilter cassettes, made of polypropylene and weighing 0.2 grams, which are used in aquaculture systems (Figure 5a) were treated with either standard corona-based plasma or vacuum plasma to activate before the addition of phages. For the corona-based treatment a standard belt-fed machine was used. The electrode height was set to 11mm to allow the filter units to pass under and the belt speed was set to 1 m/min. Filter units were exposed to 2 different power settings 200w, 400w on the entire outer surface of the unit. Filter units were also passed through the electrode without any corona treatment as controls. For vacuum plasma application, the vacuum plasma machine was set to 200W and the filters were treated for 8 mins. Purified Phage T4 of concentration 3x10⁹ PFU/ mL was applied. Approximately 1 minute after treatment, the filter unit was immersed in 1mL of phage solution for 30 mins at room temperature. All filter units were dried in a biosafety cabinet for 16h at room temperature.

Phage activity was assessed before drying (day 0) immediately after drying (day 1) and after 7 days incubation at 35°C. As shown in Figure 5b, at day 7, vacuum plasma treated filters had approximately 10x more activity than corona-treated filters and >100 times more activity than those which had no treatment.

Thus, the invention provides a method of covalently attaching biological macromolecule to a substrate comprising the steps of: (a) generating a plasma at a location separated from the substrate; and (b) contacting the substrate exposed to the plasma with a biological macromolecule. The invention also provides The invention also provides an apparatus comprising covalently attaching a biological macromolecule to a substrate comprising: (a) a treatment area or chamber; (b) means for generating a plasma at a location separate from the treatment area or chamber; (c) means for contacting objects in the treatment area or chamber with the biological macromolecule. Methods and apparatus of the invention thus enable the application of one or more of the following features: the plasma used for activation being initially generated at a site distinct from the object being treated; a biological macromolecule being applied to the substrate by application of carrier fluid, such as a liquid or gas; the biological macromolecule being applied to the surface rapidly (< 1 minute) after plasma activation to enable immobilisation (i.e. covalent attachment) of, preferably, bacteriophages; the biological macromolecule retaining complete or partial activity after immobilisation.

### References

[1] C. Wang, D. Sauvageau, A. Elias Immobilization of Active Bacteriophages on Polyhydroxyalkanoate Surfaces. ACS Appl. Mater. Interfaces 2016, 8, 1128-1138
[2] Barton, D.; Short, R. D.; Fraser, S.; Bradley, J. W. The Effect of Ion Energy upon Plasma Polymerization Deposition Rate for Acrylic Acid. Chem. Commun. (Cambridge, U. K) 2003, 7, 348-349.
[3] Canavan, H. E.; Cheng, X.; Graham, D. J.; Ratner, B. D.; Castner, D. G. A Plasma-Deposited Surface for Cell Sheet Engineering: Advantages over Mechanical Dissociation of Cells. Plasma Processes Polym. 2006, 3, 516-523.
[4] Recek, N.; Mozetic, M.; Jaganjac, M.; Milkovic, L.; Zarkovic, N.; Vesel, A. Adsorption of Proteins and Cell Adhesion to Plasma Treated Polymer Substrates. Int. J. Polym. Mater. 2014, 63, 685-691.
[5] Kerkeni, A.; Behary, N.; Dhulster, P.; Chihib, N.; Perwuelz, A. Study on the Effect of Plasma Treatment of Woven Polyester Fabrics with Respect to Nisin Adsorption and Antibacterial Activity. J. Appl. Polym. Sci. 2013, 129, 866-873.
[6] Hirsh, S. L., Bilek, M. M. M., Nosworthy, N. J., Kondyurin, A., Dos Remedios, C. G., & McKenzie, D. R. (2010). A comparison of covalent immobilization and physical adsorption of a cellulase enzyme mixture. Langmuir, 26 (17), 14380-14388.

## Claims

1. A method of covalently attaching a biological macromolecule to a substrate comprising:
(a) generating a plasma at a location separated from the substrate;
(b) contacting the substrate exposed to the plasma with the biological macromolecule.

2. A method according to claim 1, comprising an additional step of moving the plasma to expose the substrate thereto.

3. A method according to claim 1 or claim 2, wherein the biological macromolecule contacts the substrate under conditions of reduced pressure or vacuum.

4. A method according to any of claims 1 to 3, wherein the plasma generated is moved by a fluid to expose the substrate to the plasma.

5. A method according to any preceding claim, wherein the biological macromolecule is a bacteriophage, preferably the bacteriophage retains infectivity.

6. A method according to any preceding claim, wherein the exposing step is carried out within a chamber.

7. A method according to any previous claim, wherein the biological macromolecules are attached on substantially all surfaces of the substrate.

8. A method according to any preceding claim, wherein the substrate is a powder.

9. A method according to claim 8, comprising the step of locating the powder in a rotatable drum.

10. A method according to any of claims 1 to 9, wherein the substrate is a textile.

11. A method according to any of claims 1 to 9, wherein the substrate is paper.

12. A method according to any of claims 1 to 11, wherein the substrate is a medical device.

13. An apparatus for covalently attaching a biological macromolecule to a substrate comprising:
(a) a treatment area or chamber;
(b) means for generating a plasma at a location separate from the treatment area or chamber; and
(c) means for contacting objects in the treatment area or chamber with the biological macromolecule.

14. An apparatus according to claim 13, wherein the apparatus additionally comprises means for moving the plasma to the treatment area or chamber.

15. An apparatus according to claim 13 or 14, wherein the treatment area or chamber comprises a rotatable drum, preferably the rotatable drum is removable from the apparatus.
